# FASCICULE DE BREVET EUROPEEN

(11) **EP 4 138 996 B1**
(45) Date de publication et mention de la délivrance du brevet: **12.08.2026**
(21) Numéro de dépôt: 21720289.4
(22) Date de dépôt: 23.04.2021
(51) Int. Cl.: A61N 5/06

(54) **DISPOSITIF DE PHOTOTHÉRAPIE**
LICHTTHERAPIEVORRICHTUNG
PHOTOTHERAPY DEVICE

(30) Priorité: 23.04.2020 EP 20305398
(43) Date de publication de la demande: 01.03.2023
(73) Titulaire: L'Opticreal, 49123 Ingrandes-le-Fresne-sur-Loire (FR)
(72) Inventeur: LOONES, Yves, 49123 Ingrandes-le-Fresne-sur-Loire (FR)
(74) Mandataire: Icosa
(86) Numéro de dépôt international: PCT/EP2021/060616
(87) Numéro de publication internationale: WO 2021/214268

(56) Documents cités:
- KR-B1- 100 818 550
- US-A1- 2007 167 998
- US-A1- 2012 310 309

## Description

### DOMAINE DE L'INVENTION

La présente invention concerne un dispositif de photothérapie utilisant de la lumière monochromatique polarisée circulairement.

### ÉTAT DE LA TECHNIQUE

La photothérapie est une technique reposant sur l'exposition d'une partie du corps humain à la lumière pour induire des effets bénéfiques, notamment pour des applications médicales, paramédicales, physio-thérapeutiques, énergétiques ou esthétiques.

Dans ce domaine, on connait la demande WO200628461A2 concernant un procédé et un système pour l'éclairage d'un élément du corps humain comportant des composants qui s'adaptent autour d'un élément corporel, ou sont situés à un endroit particulier sur ou adjacent au corps humain. Dans cette invention, l'élément corporel est exposé à une lumière ayant une longueur d'onde à l'intérieur d'une première plage de longueurs d'onde et ensuite à une lumière ayant une longueur d'onde à l'intérieur d'une deuxième plage de longueurs. Cette invention ne précise pas la largeur des première et deuxième plages de longueurs d'onde. En outre, cet art antérieur ne comporte aucun moyen pour polariser la lumière.

On connait également la demande EP2243513A1 concernant une méthode et un dispositif de photothérapie conçu pour correspondre aux détails anatomiques du corps humain afin d'exposer simultanément des régions de la surface du corps humain à la lumière. Bien que ce dispositif puisse être conçu pour transmettre une lumière avec des caractéristiques de fréquence et polarisation spécifiques, cet art antérieur ne traite pas d'une lumière polarisée circulaire dans le domaine de la photothérapie.

On connait aussi la demande EP1715918 concernant un appareil de photothérapie comportant une source de lumière, un guide de lumière adapté à amener la lumière en entrée d'un stylet pour projeter un faisceau lumineux sur des tissus vivants et comportant une lame en sortie d'un polariseur agencée pour imposer à la lumière un sens (D, L) de polarisation déterminé.

Enfin, on connait la demande KR100818550 concernant un appareil laser de contact avec le cuir chevelu en forme de stylo et conçu pour faciliter le traitement de croissance capillaire sans endommager les cheveux, en irradiant des faisceaux laser directement sur le cuir chevelu. Cet appareil comprend un laser, une fibre optique, un polariseur convertissant le faisceau laser parallèle en lumière polarisée linéaire, et un dispositif de retard de phase convertissant la lumière polarisée linéaire en lumière polarisée circulaire. La présente invention a pour objet de palier les inconvénients de l'art antérieur et d'augmenter l'efficacité de la photothérapie. Pour cela, le but de l'invention est un dispositif de photothérapie pour délivrer une lumière polarisée circulaire ayant un taux d'ellipticité très faible, afin d'obtenir une performance améliorée dans les traitements médicaux, paramédicaux, physio-thérapeutiques, énergétiques ou esthétiques reposant sur l'utilisation de la lumière.

### RÉSUMÉ

La présente invention concerne un dispositif de photothérapie tel que défini dans les revendications attenantes.

Par construction, ce dispositif délivre une lumière polarisée circulairement.

Dans un mode de réalisation, la source de lumière comprend un générateur de lumière et une optique de collimation du faisceau lumineux pour collimater la lumière en sortie du générateur.

Selon un mode de réalisation, le générateur de lumière du dispositif selon l'invention est une source de lumière portable intégrée dans le stylet, par exemple une source de lumière LED. L'utilisation d'une source de lumière portable intégrée dans le stylet permet de s'affranchir des câbles et d'obtenir un dispositif peu bruyant. Alternativement, le générateur de lumière du dispositif est un générateur relié au stylet via un câble à fibre optique. De tels générateurs, reliés au stylet via un câble à fibre optique, sont des générateurs de lumières standard, diffusés dans le domaine médical et peuvent être des générateurs utilisant une lampe xénon ou des générateurs laser de faible ou haute intensité émettant de la lumière laser de basse ou haute puissance.

Dans un mode de réalisation de la présente invention, le bloc optique est un embout interchangeable. Cela assure la flexibilité et versatilité du dispositif, qui peut être rapidement adaptées à des applications différentes en changeant le bloc optique et, par conséquent, les caractéristiques de longueur d'onde et de sens de polarisation de la lumière polarisée circulaire transmise.

Dans la présente invention, la lumière polarisée circulairement présente une ellipticité inférieure ou égale à 3%. Ce taux d'ellipticité très faible est associé à une performance améliorée dans les traitements médicales, physio-thérapeutiques, paramédicales ou esthétiques reposant sur l'utilisation de la lumière.

De préférence, la longueur d'onde λ₀ est comprise entre 400 nm et 700 nm afin de transmettre une lumière ayant longueur d'onde dans la plage du visible en évitant les effets indésirables liés aux rayonnements infrarouge et ultraviolet.

Dans un mode de réalisation de la présente invention, la longueur d'onde λ₀ de la lumière est choisie parmi les suivantes : 400 ou 440 nm ou 700 nm, et la lumière est polarisée circulairement à gauche, ce qui est particulièrement adapté au traitement des affections traumatiques des tissus osseux et tendino-ligamentaires.

Dans un mode de réalisation de la présente invention, la longueur d'onde λ₀ de la lumière est choisie parmi les suivantes : 400 ou 440 nm ou 700 nm; et a lumière est polarisée circulairement à droite, ce qui est particulièrement adapté au traitement des affections inflammatoire post-traumatiques des tissus osseux et tendino-ligamentaires.

Dans un mode de réalisation de la présente invention, la longueur d'onde λ₀ de la lumière est choisie entre 400 et 550 nm ou entre 550 nm et 700 nm, et la lumière est polarisée circulairement à droite ou gauche ; ce qui est particulièrement adapté à la régulation de l'équilibre acido-basique et/ou la régulation de l'équilibre d'oxydo-réduction.

Dans un mode de réalisation de la présente invention, la longueur d'onde λ₀ de la lumière est choisie parmi les suivantes : 400 nm, 470 nm, 500 nm, 616 nm ou 694 nm ; ce qui est particulièrement adapté au traitement de la douleur.

Dans un mode de réalisation du dispositif selon la présente invention, la lumière est polarisée circulairement à gauche et de longueur d'onde λ₀ choisie parmi les suivantes : 400 nm, 420 nm, 440 nm, 470 nm, 500 nm ou 700 nm ; ce qui est particulièrement adapté au domaine esthétique de l'anti-âge.

La présente invention concerne également un dispositif pour l'application de la lumière dans les réseaux énergétiques et les points d'acupuncture des méridiens décrits dans la médecine traditionnelle chinoise.

### DÉFINITIONS

Dans la présente invention, les termes ci-dessous sont définis de la manière suivante :
- Le **"potentiel hydrogène"** ou **"pH"** est l'opposé du logarithme décimal de la concentration molaire en ions hydronium H₃O⁺. Il est compris entre 0 et 14.
- Le **"potentiel en dihydrogène"** ou **"rH2"** est l'opposé du logarithme décimal de l'activité de l'hydrogène moléculaire H₂. Il est compris entre 0 et 42.
- **L'"ellipticité"** est le rapport (*Imax-Imin*) / *Imax* exprimé en pourcentage ; *Imax* et *Imin* étant respectivement la valeur maximale et la valeur minimale de la puissance optique de la lumière, mesurée à l'aide d'un polarimètre.

### DESCRIPTION DÉTAILLÉE

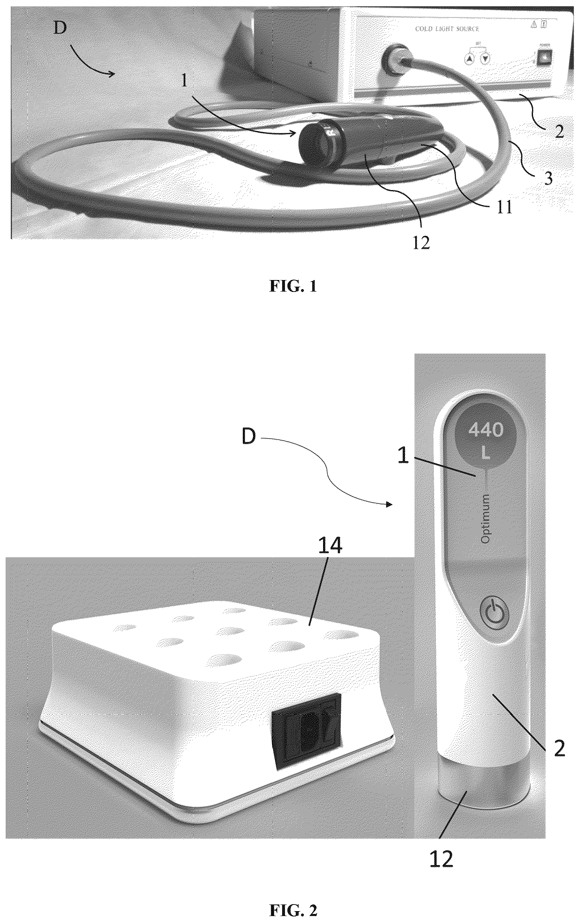

La présente invention concerne un dispositif de photothérapie utilisant de la lumière monochromatique polarisée circulairement et elle sera mieux comprise à la lecture de la **figure 1** qui illustre non-limitativement l'invention.

Comme illustré dans la **figure 1****,** le dispositif D comprend une source de lumière et un stylet 1 comprenant un corps de stylet 11 et un bloc optique (non visible). Dans un mode de réalisation, la source de lumière comprend un générateur de lumière 2 et une optique de collimation du faisceau lumineux. Dans le mode de réalisation représenté en **figure 1****,** le générateur de lumière 2 est un générateur xénon relié au stylet via un câble à fibre optique 3. En variante représentée en **figure 2****,** le générateur de lumière 2 est une source de lumière LED portable intégrée dans le stylet. Dans cette variante, le stylet 1 comprend une batterie, de préférence une batterie rechargeable.

Dans le mode de réalisation représenté en **figure 1****,** le bloc optique est compris dans un embout interchangeable 12. Dans un mode de réalisation alternatif non représenté, le bloc optique est intégré dans le stylet 1.

Le bloc optique comprend un filtre interférentiel, un polariseur et une lame quart d'onde. Le filtre interférentiel est de type passe-bande à bande passante étroite, centré sur une longueur d'onde λ₀ avec une précision de ±2 nm et ayant une largeur à mi-hauteur inférieure ou égale à 10 nm. L'interposition du filtre interférentiel sur le trajet du faisceau lumineux permet de bloquer ou d'atténuer fortement toutes les radiations lumineuses sauf celles appartenant à la bande passante étroite caractéristique du filtre. Le polariseur et la lame quart d'onde sont agencés en succession dans la direction de propagation du faisceau lumineux émis par la source de lumière, ce qui permet d'obtenir une lumière polarisée circulairement. Le filtre interférentiel est agencé avant le polariseur et la lame quart d'onde ou après le polariseur et la lame quart d'onde. De préférence, le filtre interférentiel est agencé avant le polariseur et la lame quart d'onde afin de ne pas modifier la polarisation de la lumière. Le polariseur ne laisse passer que la composante de la radiation lumineuse incidente ayant une polarisation parallèle à l'axe caractéristique du polariseur. La lumière sortant du polariseur est ainsi polarisée rectilignement. La lame quart d'onde selon la présente invention est une lame retardatrice en quartz d'ordre zéro apportant un retard (déphasage) entre les deux composantes du champ électrique de la lumière incidente selon les deux axes de ladite lame. Le retard apporté par ladite lame quart d'onde est égal à un quart de λ₀ pour une radiation incidente ayant longueur d'onde λ₀, de manière à transformer la lumière polarisée rectilignement incidente en une lumière polarisée circulairement. Dans la présente invention, par lumière polarisée circulairement on entend une lumière dans laquelle l'extrémité du vecteur champ électrique décrit une trajectoire elliptique dans un plan perpendiculaire à la direction de propagation de ladite lumière. Du fait de l'utilisation d'un filtre à bande passante étroite centré sur la longueur d'onde λ₀, la trajectoire elliptique est proche d'un cercle. La trajectoire elliptique est une ellipse avec une ellipticité très faible, inférieure ou égale à 3%.

Par sens de polarisation on entend le sens de parcours de ladite trajectoire elliptique par le vecteur champ électrique, le sens étant droit ou gauche en fonction du positionnement des deux axes de la lame quart d'onde.

L'ellipticité de la lumière polarisée circulaire dans la présente invention est calculée à l'aide d'un polarimètre comprenant une lame retardatrice tournante de la façon suivante : le polarimètre est illuminé avec le dispositif D de photothérapie selon la présente invention ; plusieurs mesures de puissance optique de la lumière atteignant le polarimètre sont prises en faisant varier l'angle de la lame retardatrice tournante ; l'ellipticité est calculée comme le rapport *(Imax-Imin)* / *Imax* exprimé en pourcentage ; *Imax* et *Imin* étant respectivement la valeur maximale et la valeur minimale ou les deux valeurs orthogonales de la puissance optique mesurée. L'ellipticité est comprise entre 0% et 100%, le premier cas (0%) correspondant à une lumière ayant polarisation parfaitement circulaire et le second cas (100%) correspondant à une lumière ayant une polarisation parfaitement rectiligne.

De manière avantageuse, le dispositif D selon l'invention comprend un embout interchangeable 12. Dans ce mode de réalisation, chaque embout interchangeable 12 comprend un bloc optique agencé pour transmettre une lumière ayant une longueur d'onde λ₀ différente et une polarisation circulaire droite ou gauche. Cette configuration permet de changer avec une opération simple et rapide les caractéristiques de longueur d'onde et sens de polarisation de la lumière polarisée circulaire transmise par le dispositif D, afin de s'adapter à des applications différentes. Par applications différentes, on entend des applications de la photothérapie à différentes conditions pathologiques ou physiologiques.

Avantageusement, le stylet 11 du dispositif D selon la présente invention est positionné à proximité de la partie du corps humain que l'on souhaite exposer à la lumière polarisée circulaire à l'aide d'un support de stylet. Le support de stylet comprend un support coulissant, un flexible de positionnement du stylet, et un pince stylet. Plusieurs supports de stylet sont envisageables afin d'exposer différentes parties du corps humaine à la lumière polarisée circulaire simultanément.

En général, la longueur d'onde λ₀ est comprise entre 400 et 700 nm. De préférence, λ₀ est égale *- i.e.* égale à plus ou moins 2 nm - à une des longueurs d'onde suivantes : 400 nm, 420 nm, 440 nm, 470 nm, 484 nm, 500 nm, 520nm, 532 nm, 550nm, 568 nm, 580 nm, 600 nm, 616 nm, 630 nm, 660 nm, 694 nm, 700 nm.

Pour chaque longueur d'onde, le sens droit ou gauche de la lumière polarisée circulairement est sélectionné en fonction de la condition que l'on souhaite traiter. A titre d'exemple, dans le cadre du traitement de la glande thyroïde, une longueur d'onde λ₀ égale à 616 nm est préférable ; dans ce cas particulier, la lumière est polarisée circulaire droite en présence d'un taux élevée d'hormones thyroïdiennes et polarisée circulaire gauche en présence d'un taux faible d'hormones thyroïdiennes.

Dans un mode de réalisation de la présente invention, la longueur d'onde λ₀ de la lumière est choisie parmi les suivantes : 400 nm, 470 nm, 500 nm, 616 nm, 694 nm ou 700 nm, ce qui est particulièrement adapté au traitement de la douleur.

Dans le traitement des douleurs aiguës et chroniques, la longueur d'onde de la lumière polarisée circulaire est choisie parmi les suivantes : 400 nm, 470 nm, 500 nm, 616 nm, 694 nm ou 700 nm. De préférence, λ₀ est égale à 616 nm ou 694 nm en cas de douleur due aux bursites et/ou tendinites ; alors que λ₀ est de préférence 500 nm ou 616 nm en cas de douleurs prémenstruelles.

Dans un cas particulier, le dispositif D de photothérapie selon la présente invention est un dispositif de photothérapie adapté pour réguler des paramètres bioélectroniques tels que le potentiel hydrogène (pH), le potentiel en dihydrogène (rH₂), et la résistivité électrique (ρ). La régulation des paramètres bioélectroniques comprend la régulation de l'équilibre acido-basique via alcalinisation (augmentation des valeurs de pH) ou acidification (diminution des valeurs de pH) et la régulation de l'équilibre d'oxydo-réduction via oxydation (augmentation des valeurs de rH2) ou réduction (diminution des valeurs de rH2). Dans ce cas particulier, le dispositif D selon la présente invention est conçu pour une application (i) d'une lumière polarisée circulairement à droite et ayant une λ₀ comprise entre 400 et 550 nm en présence d'une valeur de pH entre 6.2 et 7.2 et une valeur de rH2 entre 15 et 22 ; et/ou (ii) d'une lumière polarisée circulairement à droite et ayant une λ₀ comprise entre 550 nm et 700 nm en présence d'une valeur de pH entre 6.2 et 7.2 et une valeur de rH2 entre 22 et 33 ; et/ou (iii) d'une lumière polarisée circulairement à gauche et ayant une λ₀ comprise entre 400 nm et 550 nm en présence d'une valeur de pH entre 7.2 et 9.4 et une valeur de rH2 entre 15 et 22 ; et/ou (iv) d'une lumière polarisée circulairement à gauche et ayant une λ₀ comprise entre 550 nm et 700 nm en présence d'une valeur de pH entre 7.2 et 9.4 et une valeur de rH2 entre 22 et 33.

Dans un autre cas particulier, le dispositif D selon la présente invention est pour une application esthétique. On entend par applications esthétiques, par exemple le traitement des signes du vieillissement de la peau, par exemple dus à l'exposition rayons ultraviolets. Dans ce cas, la lumière transmise par le dispositif D est polarisée circulaire à gauche et la longueur d'onde λ₀ est choisie parmi les suivantes : à 400 nm, 420 nm, 440 nm, 470 nm, 500 nm ou 700 nm.

Par signes du vieillissement de la peau, on entend notamment un facteur ou une combinaison de facteurs tels que, mais non limités à, une diminution du pourcentage de collagène dans le derme, une diminution du pourcentage et/ou la longueur des fibres oxytalanes et élaunines, une diminution de glycosaminoglycanes, une augmentation de la concentration de radicaux libres, une augmentation de l'expression des métalloprotéinases matricielles (MMP) telles que la MMP-1.

De préférence, la lumière polarisée circulairement délivrée par le dispositif de la présente invention est une lumière polarisée circulaire gauche ayant longueur d'onde λ₀ choisie parmi 400 nm, 440 nm et 700 nm, pour une utilisation dans le traitement des affections traumatiques des tissus osseux et tendino-ligamentaires.

Dans d'autres cas particuliers, le bloc optique du dispositif D selon l'invention est agencé pour transmettre une lumière polarisée circulairement à gauche et ayant une longueur d'onde choisie parmi les valeurs spécifiées entre parenthèses :
- application de la lumière aux affections du tissus musculo-aponévrotique (440 nm, 470 nm 500 nm ou 580 nm) ;
- application de la lumière dans les affections du tissu conjonctif (500 nm ou 700 nm) ;
- application de la lumière dans les algodystrophies et les syndromes myofasciaux (400 nm, 440 nm, 470 nm, 500 nm, 580 nm, 616 nm ou 700 nm) ;
- application de la lumière en cas de lombalgie et cervicalgie hors étiologie inflammatoire (400 nm, 520 nm, 580 nm, 616 nm ou 700) ;
- application de la lumière dans le cadre du traitement des hypothyroïdies (400nm, 440 nm,616 nm, 660 nm ou 700 nm) ; et
- application de la lumière dans le traitement des syndromes métaboliques et affections hépatiques (500nm, 520nm, 532 nm ou 550nm).

Alternativement, la lumière polarisée circulairement délivrée par le dispositif de la présente invention est une lumière polarisée circulaire droite ayant longueur d'onde λ₀ choisie parmi 400 nm, 440 nm et 700 nm, pour une utilisation dans le traitement des affections inflammatoires post traumatiques des tissus osseux et tendino-ligamentaires.

Dans d'autres cas particuliers, le bloc optique du dispositif D selon l'invention est agencé pour transmettre une lumière polarisée circulairement à droite et ayant une longueur d'onde λ₀ choisie parmi les valeurs spécifiées entre parenthèses :
- application de la lumière dans le traitement des allergies - dégranulation des mastocytes (568 nm ou 580 nm) ;
- application de la lumière dans les affections inflammatoires du tendino-ligamentaires (600 nm, 616 nm ou 660 nm) ;
- application de la lumière dans le traitement des algodystrophies (580 nm, 616 nm, 660 nm ou 700 nm) ;
- application de la lumière aux hyperthyroïdies (600 nm, 616 nm, 660 nm ou 700 nm);
- application de la lumière dans les affections virales - herpès, zona, grippe, dingue (580 nm ou 600 nm) ;
- application de la lumière dans les affections de sur-électronisation positive (600 nm);
- application de la lumière dans les affections cardio-vasculaires, les affections pancréatique , les diabètes, les déséquilibres hormonaux, les syndromes inflammatoires et les brûlures (440 nm, 500 nm, 580 nm, 600 nm, 616 nm ou 660 nm).

La présente invention concerne également un dispositif pour l'application de la lumière polarisée circulairement en correspondance des réseaux énergétiques et en correspondance des points d'acupuncture des méridiens principaux tels que décrits dans la médecine traditionnelle chinoise. Dans ce cas, λ₀ est égale à : 400 nm pour une application dans le méridien du rein ; 440 nm pour une application dans le méridien des poumons ; 500 nm pour une application dans le méridien du maitre cœur ; 600 nm pour une application dans le méridien du triple réchauffeur ; 616 nm pour une application dans le méridien du cœur, rate et pancréas ; 660 nm pour une application dans le méridien de l'estomac et du gros intestin ; 700 nm pour une application dans le méridien de vessie et intestin grêle. Enfin, pour une application dans le méridien du foie et/ou dans le méridien de la vésicule biliaire, λ₀ est choisi parmi 500 nm, 520 nm, 532 nm et 550 nm ou parmi 550 nm, 568 nm et 580 nm, respectivement. Dans le dispositif pour une applications de la lumière polarisée circulairement aux méridiens, la lumière est une lumière polarisée circulairement à gauche dans le cas d'une application de la lumière aux branches gauches desdits méridiens, ou une lumière polarisée circulairement à droite dans le cas d'une application de la lumière aux branches droites desdits méridiens. Ce dispositif de photothérapie pour une application dans la stimulation des méridiens principaux est adaptable pour d'autre applications dans le domaine de la médicine traditionnelle chinoise, comme par exemple la stimulation des merveilleux vaisseaux dont tous les points de leurs trajets répondent aux mêmes λ que les méridiens principaux, de même que leurs points d'ouverture, de stimulation, ou de régulation.

Aucun effet thermique n'est associé à l'utilisation du présent dispositif D de photothérapie lorsque la puissance lumineuse de la lumière transmise par le présent dispositif D est inférieure à 1 milliwatt.

### BRÈVE DESCRIPTION DES FIGURES

La **figure 1** est une photographie montrant un mode de réalisation du dispositif (D) selon l'invention, dans lequel la source de lumière est un générateur xénon (2) relié au stylet (1) via un câble à fibre optique (3), et le bloc optique est un embout interchangeable (12) ajusté sur le corps de stylet (11).
La **figure 2** est une représentation montrant un mode de réalisation du dispositif (D) selon l'invention, dans lequel la source de lumière est une LED (2) incorporée dans le stylet (1), et le bloc optique (12) est ajusté sur le stylet (1). Une batterie rechargeable est comprise dans le stylet. Un dock de recharge (14) permet de recharger la batterie incluse dans le stylet. Le dock de recharge (14) présenté ici permet de recharger simultanément 9 stylets.

### EXEMPLES

La présente invention se comprendra mieux à la lecture des exemples suivants qui illustrent non-limitativement l'invention.

### Exemple 1 : évaluation de l'efficacité du dispositif de photothérapie dans la diminution de la douleur chez les patients.

### Matériel et Méthodes

L'étude suivante est réalisée pour comparer la douleur, quantifiée via l'Echelle Visuelle Analogique (EVA) de la douleur, chez les patients avant et après exposition à la lumière polarisée circulaire transmise pas le dispositif selon la présente invention. La douleur est quantifiée à l'aide de l'EVA de la manière suivante :
- Absence de douleur : EVA égal à 0
- Douleur légère : EVA compris entre 1 et 3
- Douleur modérée : EVA compris entre 4 et 7
- Douleur sévère : EVA compris entre 8 et 10

En particulier dans cette étude 105 patients atteints de tendinites (32 patients), bursites (21 patients), ou syndromes de douleur myofasciale SDM (52 patients) ont été inclus et randomisés en trois groupes. Les longueurs d'onde λ₀ de la lumière polarisée circulaire, le nombre d'exposition et les états pathologiques des patients inclus dans l'étude sont spécifiés pour chaque groupe dans le tableau suivant :

| | Longueurs d'onde λ₀ (nm) | Nombre d'expositions à chaque λ₀ | Pathologie | | |
|---|---|---|---|---|---|
| | | | Tendinite | Bursite | SDM |
| Groupe 1 | 694 ; 616 | 2 | 20 | 16 | 0 |
| Groupe 2 | 470 ; 400 | 1 | 0 | 0 | 35 |
| Groupe 3 | / | 0 (contrôle négatif) | 12 | 5 | 17 |

### Résultats :

Pour chaque groupe, le pourcentage de patients ressentant une intensité de douleur quantifié selon l'échelle EVA avant et après exposition à lumière polarisée circulaire est indiqués dans le tableau suivant :

| | Groupe 1 | | Groupe 2 | | Groupe 3 | |
|---|---|---|---|---|---|---|
| | Avant | Après | Avant | Après | Avant | Après |
| Absence de douleur | 0 | 25 | 0 | 34.3 | 0 | 14.7 |
| Douleur légère | 0 | 44.4 | 2.9 | 40 | 5.9 | 26.5 |
| Douleur modérée | 66.7 | 27.8 | 57.1 | 22.9 | 64.7 | 44.1 |
| Douleur sévère | 33.3 | 2.8 | 40 | 2.9 | 40 | 14.7 |
| Pourcentage de patients ayant une diminution de la douleur | 86 | | 85.7 | | 41 | |

En conclusion, une diminution significative (p=0.0001) de l'intensité de douleur a été observée chez les patients exposés à la lumière polarisée circulaire via le dispositif selon l'invention (groupes 1 et 2) par rapport au groupe contrôle (groupe 3).

### Exemple 2 : évaluation de l'efficacité du dispositif de photothérapie dans l'atténuation des signes du vieillissement de la peau.

### Matériel et Méthodes

Les tests suivants sont réalisés pour évaluer les effets de la lumière polarisée circulaire sur des marqueurs de vieillissement de la peau sur un modèle expérimental de vieillissement cutané *ex vivo* basé sur le maintien en survie d'explants de peau humaine. Ces marqueurs sont mesurés sur des explants de peau de contrôle (groupe 1) ; sur des explants de peau exposés aux UV (groupe 2) ; et sur des explants de peau exposés aux UV et à la lumière polarisée circulaire du dispositif selon l'invention (groupe 3). A moins qu'il soit spécifié autrement dans le tableau ci-dessous, la longueur d'onde λ₀ de la lumière polarisée circulaire utilisée dans le groupe 3 est égale à 500 nm.

Les marqueurs immuno-histologiques de vieillissement de la peau suivants sont évalués : surface couverte par des fibres de collagène ; niveau d'expression de MMP-1 ; longueur des fibres oxytalanes et élaunines ; niveau d'expression de CD44 ; quantité de glycosaminoglycanes dans le derme superficiel et réticulaire ; niveau d'expression de Ki67.

Le marqueur biochimique de vieillissement de la peau évalué est la concentration de nitrites.

### Résultats

Les valeurs moyennes des marqueurs immuno-histologique et biochimiques pour les 3 groupes d'explants de peau sont indiqués dans le tableau suivant :

| | Groupe 1 | Groupe 2 | Groupe 3 |
|---|---|---|---|
| Collagène (% surface) (λ₀=420 nm) | 87.5 ± 4.6 | 77.75 ± 7.55 | 87.35 ± 3.85 |
| MMP-1 (valeur semi-quantitative) | 13.4 ± 5.6 | 28 ± 7.3 | 16.4 ± 9 |
| CD44 (valeur semi-quantitative) | 3.4 ± 0.35 | 1.3 ± 0.2 | 2.3 ± 0.54 |
| Longueur des fibres oxytalanes (µm) (λ₀=694 nm) | 64.4 ± 13.4 | 39.5 ± 12 | 60.4 ± 20 |
| Longueur des fibres élaunines (µm) (λ₀=694 nm) | 140.4 ± 38.1 | 102.5 ± 48.8 | 141.1 ± 40.5 |
| Glycosaminoglycanes dans le derme superficiel (valeur semi-quantitative) (λ₀=694 nm) | 1.1 ± 0.5 | 0.7 ± 0.9 | 1.4 ± 1 |
| Glycosaminoglycanes dans le derme réticulaire (valeur semi-quantitative) (λ₀=694 nm) | 1.2 ± 0.7 | 0.46 ± 0.6 | 1.5 ± 1 |
| Ki67 (valeur semi-quantitative) | 3.74 ± 2.44 | 3 ± 1.3 | 5.8 ± 3.6 |
| Nitrites (µM) | 6 ± 1.75 | 6.85 ± 1.9 | 5.6 ± 1.7 |

L'effet induit par les UV est significativement atténué suite à l'exposition à la lumière polarisée circulaire dans les marqueurs de vieillissement cutané suivants (p-valeur<0.05) : surface couverte par des fibres de collagène ; longueur des fibres oxytalanes et élaunines ; glycosaminoglycannes dans le derme réticulaire.

## Revendications

1. Un dispositif (D) de photothérapie comprenant :
a. une source de lumière et
b. un stylet (1) comprenant
i. un corps de stylet (11) et
ii. un bloc optique comprenant :
- un filtre interférentiel centré sur une longueur d'onde λ₀,
- un polariseur et
- une lame quart d'onde pour la longueur d'onde λ₀,
**caractérisé en ce que** le filtre interférentiel a une largeur à mi-hauteur inférieure ou égale à 10 nm et la lumière polarisée circulairement présente une ellipticité inférieure ou égale à 3%.

2. Dispositif selon la revendication **1,** dans lequel la source de lumière comprend un générateur de lumière (2) et une optique de collimation du faisceau lumineux.

3. Dispositif selon la revendication **2,** dans lequel générateur de lumière (2) est une source de lumière portable intégrée dans le stylet (1) ou un générateur relié au stylet (1) via un câble à fibre optique (3).

4. Dispositif selon l'une quelconque des revendications **1** à **3,** dans lequel le bloc optique est un embout interchangeable (12).

5. Dispositif selon l'une quelconque des revendications **1** à **4,** dans lequel la longueur d'onde λ₀ est comprise entre 400 nm et 700 nm.

6. Dispositif selon l'une quelconque des revendications **1** à **5,** dans lequel
a. la longueur d'onde λ₀ de la lumière est choisie parmi les suivantes : 400 ou 440 nm ou 700 nm ; et
b. la lumière est polarisée circulairement à gauche.

7. Dispositif selon l'une quelconque des revendications **1** à **5,** dans lequel
a. la longueur d'onde λ₀ de la lumière est choisie parmi les suivantes : 400 nm ou 440 nm ou 700 nm ; et
b. la lumière est polarisée circulairement à droite.

8. Dispositif selon l'une quelconques des revendications **1** à **5,** dans lequel
a) la longueur d'onde λ₀ de la lumière est choisie entre 400 et 550 nm ou entre 550 nm et 700 nm ; et
b) la lumière est polarisée circulairement à droite ou à gauche.

9. Dispositif selon l'une quelconque des revendications **1** à **5,** dans lequel la longueur d'onde λ₀ de la lumière est choisie parmi les suivantes : 400 nm, 470 nm, 500 nm, 616 nm ou 694 nm.

10. Dispositif selon l'une quelconque des revendications **1** à **5,** dans lequel la lumière est polarisée circulairement à gauche et de longueur d'onde λ₀ choisie parmi les suivantes : 400 nm, 420 nm, 440 nm, 470 nm, 500 nm ou 700 nm.

## Patentansprüche

1. Phototherapievorrichtung (D), umfassend:
a. eine Lichtquelle und
b. einen Pen (1), umfassend
i. einen Penkörper (11) und
ii. einen optischen Block, umfassend:
- einen Interferenzfilter, der auf eine Wellenlänge λ₀ zentriert ist,
- einen Polarisator und
- eine Viertelwellenplatte für die Wellenlänge λ₀,
**dadurch gekennzeichnet, dass** der Interferenzfilter eine volle Breite bei halbem Maximum kleiner oder gleich 10 nm aufweist und das kreisförmig polarisierte Licht eine Elliptizität kleiner oder gleich 3 % aufweist.

2. Vorrichtung nach Anspruch **1,** wobei die Lichtquelle einen Lichtgenerator (2) und eine Lichtstrahlkollimationsoptik umfasst.

3. Vorrichtung nach Anspruch **2,** wobei der Lichtgenerator (2) eine in den Pen (1) integrierte tragbare Lichtquelle oder ein über ein Glasfaserkabel (3) mit dem Pen (1) verbundener Generator ist.

4. Vorrichtung nach einem der Ansprüche **1** bis **3,** wobei der optische Block eine austauschbare Spitze (12) ist.

5. Vorrichtung nach einem der Ansprüche **1** bis **4,** wobei die Wellenlänge λ₀ zwischen 400 nm und 700 nm liegt.

6. Vorrichtung nach einem der Ansprüche **1** bis **5,** wobei
a. die Wellenlänge λ₀ des Lichts ausgewählt ist aus: 400 oder 440 nm oder 700 nm; und
b. das Licht ist linkszirkular polarisiert.

7. Vorrichtung nach einem der Ansprüche **1** bis **5,** wobei
a. die Wellenlänge λ₀ des Lichts ausgewählt ist aus: 400 nm ou 440 nm oder 700 nm; und
b. das Licht ist rechtszirkular polarisiert.

8. Vorrichtung nach einem der Ansprüche **1** bis **5,** wobei
a) die Wellenlänge λ₀ des Lichts zwischen 400 und 550 nm oder zwischen 550 nm und 700 nm ausgewählt ist; und
b) das Licht ist linkszirkular polarisiert.

9. Vorrichtung nach einem der Ansprüche **1** bis **5,** wobei die Wellenlänge λ₀ des Lichts aus den folgenden ausgewählt ist: 400 nm, 470 nm, 500 nm, 616 nm oder 694 nm.

10. Vorrichtung nach einem der Ansprüche **1** bis **5,** wobei das das Licht ist linkszirkular polarisiert und eine Wellenlänge λ₀ hat, die aus den folgenden ausgewählt ist: 400 nm, 420 nm, 440 nm, 470 nm, 500 nm oder 700 nm.

## Claims

1. A phototherapy device (D) comprising:
a. a light source ; and
b. a stylus (1) comprising
i. a stylus body (11) ; and
ii. an optical block comprising:
- an interference filter centered on a wavelength λ₀,
- a polarizer, and
- a quarter-wave plate for the wavelength λ₀,
**characterized in that** the interference filter has a full width at half maximum less than or equal to 10 nm and the circularly polarized light exhibits an ellipticity less than or equal to 3%.

2. Device according to claim **1,** wherein the light source comprises a light generator (2) and optics for collimating light beam.

3. Device according to claim **2,** wherein the light generator (2) is a portable light source integrated into the stylus (1) or a generator connected to the stylus (1) via a fiber optic cable (3).

4. Device according to any one of claims **1** to **3,** wherein the optical block is an interchangeable tip (12).

5. Device according to any one of claims **1** to **4,** wherein the wavelength λ₀ is ranging from 400 nm to 700 nm.

6. Device according to any one of claims **1** to **5,** wherein
a. the wavelength λ₀ of the light is selected from the following : 400 or 440 nm or 700 nm; and
b. the light is left circularly polarized.

7. Device according to any one of claims **1** to **5,** wherein
a. the wavelength λ₀ of the light is selected from the following : 400 nm or 440 nm or 700 nm; and
b. the light is right circularly polarized.

8. Device according to any one of claims **1** to **5,** wherein
a) the wavelength λ₀ of the light is selected between 400 and 550 nm or between 550 nm and 700 nm; and
b) the light is right or left circularly polarized.

9. Device according to any one of claims **1** to **5,** wherein the wavelength λ₀ of the light is selected from the following: 400 nm, 470 nm, 500 nm, 616 nm, or 694 nm.

10. Device according to any one of claims **1** to **5,** wherein the light is left circularly polarized and has a wavelength Xₒ selected from the following: 400 nm, 420 nm, 440 nm, 470 nm, 500 nm, or 700 nm.
